# EUROPEAN PATENT SPECIFICATION

(11) **EP 0 478 182 B1**
(45) Date of publication and mention of the grant of the patent: **04.01.1995**
(21) Application number: 91308369.7
(22) Date of filing: 12.09.1991
(51) Int. Cl.: A61F 13/15, D04H 13/00

(54) **A method and apparatus for forming a wad**
Verfahren und Vorrichtung zur Herstellung eines Kissens
Méthode et dispositif de fabrication d'un coussin

(30) Priority: 12.09.1990 ZA 907272
(43) Date of publication of application: 01.04.1992
(73) Proprietor: McNEIL-PPC, INC., New Brunswick, New Jersey 08903 (US)
(72) Inventor: Oatley, John Albert, East London, Cape Province (ZA); Bailey, Alan Steven, Ganubie, Cape Province (ZA)
(74) Representative: Fisher, Adrian John

(56) References cited:
- FR-A- 2 521 003
- US-A- 3 882 216
- US-A- 4 761 258

## Description

THIS INVENTION relates to wads of particulate material. It relates in particular to a method of forming a wad of particulate material and to apparatus for forming such a wad.

Absorbent pads for use in absorbing body fluids are well-known. Such pads include baby diapers, sanitary napkins, incontinence pads, wound dressings, breast pads for nursing mothers and the like. They are commonly made of absorbent cellulosic pulp fibres in the form of a batt or wad sandwiched between at least two layers of material. One layer is fluid-permeable and forms the layer adapted to face the wearer's body. The other layer conveniently is liquid-impervious and forms the side which faces away from the person's body and protects the person's clothing from soiling. This invention thus more specifically relates to a method and apparatus for forming a wad of particulate material suitable for use as a component of such an absorbent pad.

According to Claim 1 there is provided a method of forming a wad of particulate material, the wad having a predetermined shape, which includes
providing a permeable carrier which has first and second opposed surfaces;
providing a forming screen which also has first and second opposed surfaces and which is substantially impermeable except for a forming zone which has the said shape and which is permeable;
positioning the carrier and the screen adjacent one another with the second surface of the carrier adjacent the first surface of the screen;
generating a pressure differential across the carrier and the screen, there being a higher pressure at the first surface of the carrier such that a stream of fluid is caused to flow through the carrier substantially only in an area defined by the forming zone;
introducing the particulate material into the space adjacent the first surface of the carrier, which particulate material is carried by the fluid stream and is deposited on the first surface of the carrier; and
separating the carrier and the screen.

Further, according to Claim 13 there is provided an apparatus for forming a wad of particulate material, the wad having a predetermined shape, which includes
a forming screen which has first and second opposed surfaces and which is substantially impermeable except for a forming zone which has the said shape and which is permeable;
a supply means for supplying a permeable carrier, which also has first and second opposed surfaces, with the second surface of the carrier adjacent the first surface of the screen;
a presssure differential generating means for generating, in use, a pressure differential across the carrier and the screen, there being a higher pressure at the first surface of the carrier, such that a stream of fluid is caused to flow through the carrier substantially only in an area defined by the forming zone;
introducing means for introducing the particulate material, in use, into the space adjacent the first surface of the carrier, such that the particulate material is carried by the fluid stream to be deposited on the first surface of the carrier; and
separating means for separating, in use, the carrier and the screen.

A method and apparatus as specified in the preamble of Claims 1 and 13, respectively, are disclosed in FR-A-2521 003. The apparatus may include the carrier.

The forming zone may be an aperture in the screen, so that the whole forming zone has the same permeability. Instead, the forming zone may have a region of substantially high permeability and a region of intermediate permeability, with more particulate material being deposited on an area of the carrier in alignment with the high permeability region and less being deposited on an area in alignment with the intermediate permeability region. With such an embodiment, the forming zone may have an opening that is fairly large and which defines the region of high permeability with the region of low permeability being defined by a number of fairly small holes.

The screen may have a plurality of forming zones that are spaced apart, and the carrier and the screen may be moved together through a pressurizing and introducing station where the fluid stream is caused to flow through the carrier only in those areas that are aligned with forming zones, thereby depositing the particulate material on the carrier and successively forming the wads thereon. The apparatus may thus have a suitable moving means for moving the carrier and screen. Preferably the carrier and the screen are moved continuously through the pressurizing and introducing station. It will be appreciated that a plurality of forming zones may be located together in the pressurizing and introducing station at the same time.

The carrier is preferably a strip of non-woven fabric.

The particulate material may be fibrous and may be of a cellulosic material such as wood pulp, cotton, rayon or the like. Non-cellulosic materials may also be used such as polyolefinic or polyester fibres, etc.

The screen may be endless and may comprise a cylindrical wall of a drum or a belt which is supported, in the pressurizing and introducing station, by a drum having a sieve-like cylindrical wall.

Preferably, in the pressurizing and introducing station the carrier and screen are in contact with one another.

The pressure differential generating means may comprise a vacuum generating means located adjacent the second surface of the screen.

The apparatus may also include a shredder for shredding a sheet of fibrous material.

A wad of particulate material which has been formed using the method, or by apparatus, in accordance with the invention is protected according to Art. 64(2)EPC.

Such a wad may be stabilised and bonded to the carrier and a liquid impervious backing may be laminated to the wad and the carrier, to provide a panty liner or the like. Instead, the wad and carrier can be applied over an absorbent layer, eg. a peatmoss layer, as a transition layer, with the absorbent layer being laminated to a liquid impervious backing to form a sanitary pad or the like. In the case where the wad is sandwiched directly between the carrier and the backing, it can be relatively thick, eg. it may have a basis weight of the order of about 120 grams/m², while, when it is used as a transitiion layer, it is envisaged that it will have a basis weight of about 35 grams/m².

By means of the invention a simple method, and an apparatus that is easy to operate, are provided. The apparatus further has a simple forming station and, because the wad is not removed or transferred from one carrier to another after it is formed it is possible to produce the wads efficiently in multiple lanes.

The invention is now described by way of example with reference to the accompanying diagrammatic drawings.

In the drawings,
FIGURE 1 shows a side view of apparatus according to one embodiment of the invention, for producing wads of particulate material;
FIGURE 2 shows a side view of apparatus according to another embodiment of the invention, for producing wads of particulate material; and
FIGURE 3 shows in greater detail a portion of a forming screen of the apparatus of Figures 1 and 2;
FIGURE 4 shows a perspective view of a wad formed with a forming screen as shown in Figure 3; and
FIGURES 5 and 6 show plan views of portions of carrier strips carrying a number of wads as produced by the apparatus of Figure 1 or Figure 2.

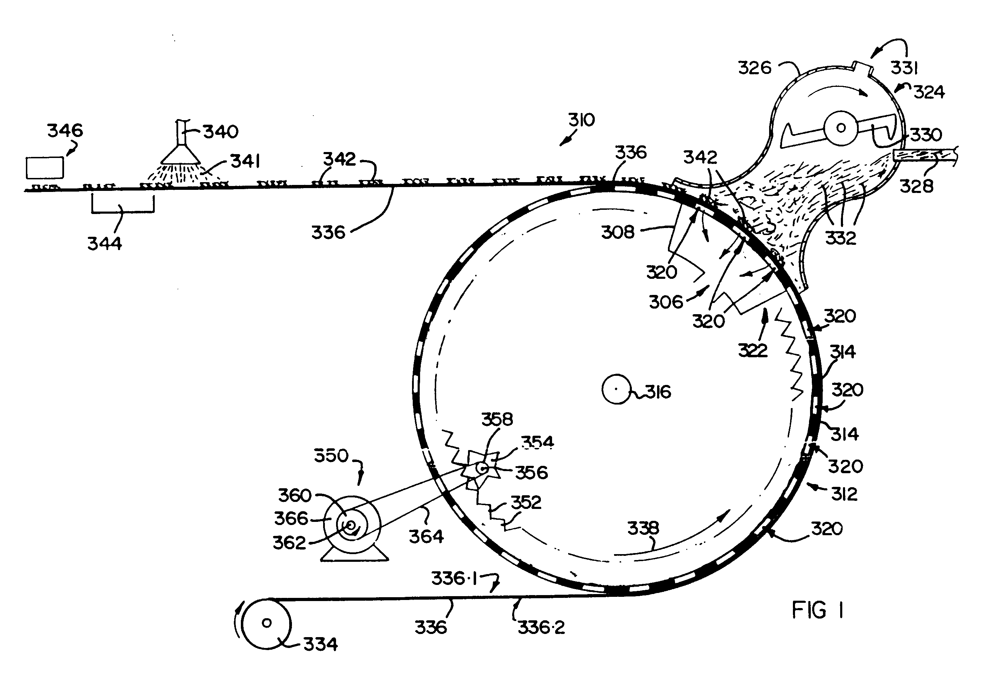

Referring to Figure 1, reference numeral 310 generally indicates apparatus according to one embodiment of the invention, for producing wads of particulate material.

The apparatus 310 includes a circular cylindrical drum, generally indicated by reference numeral 312. The drum 312 has a cylindrical wall 314. The drum 312 is adapted to be driven about a rotational axis 316 by means of suitable drive means generally indicated by reference numeral 350. The drive means 350 comprises a plurality of radially inwardly protruding circumferentially spaced teeth 352 on the drum 312. Two sets of teeth 352, located respectively at the ends of the drum 312, areprovided. The drum 312 is rotatably supported by two side plates (not shown) which have raised circular portions (also not shown) on their inner surfaces. The internal diameter of these portions is slightly larger than the outer diameter of the drum and are lined with a suitable non-slip material or with bearings. With each set of teeth 352 is associated a toothed cog 354, with the cogs 354 being mounted on a single shaft or axle 356. To one end of the shaft 356 is mounted a pulley 358 which is aligned with a pulley 360 mounted to the output shaft 362 of an electric motor 366. An endless belt 364 connects the pulleys 358, 360.

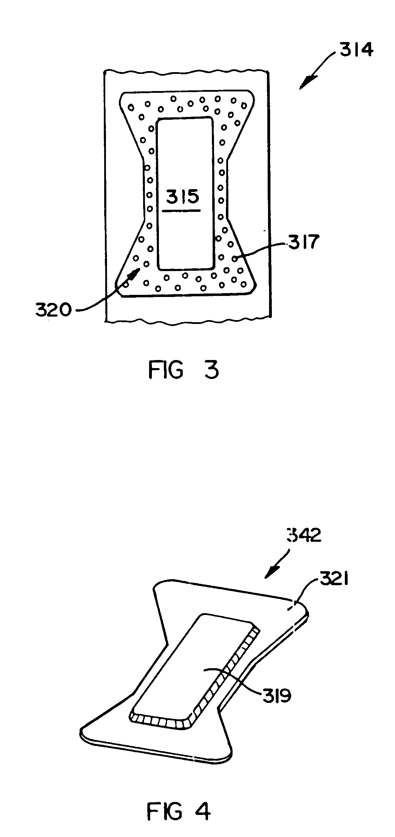

The cylindrical wall 314 of the drum 312 is generally imperforate, (ie. solid) with forming zones 320 in the form of apertures of predetermined shape, being provided therein. The apertures 320 are described in more detail below with reference to Figure 3.

The apparatus 310 also includes a vacuum generating means for applying a vacuum to the inside of the drum wall 314, at a forming station 322. The vacuum generating means includes a vacuum box 308 with a duct 306 communicating with a vacuum source (not shown). The box 308 is located within the drum 312, in contact with its wall 314. A pressure differential is formed across the drum wall with a higher pressure being present on the outside and a lower pressure on the inside.

The apparatus 310 also includes a supply means, generally indicated by reference numeral 324, for supplying shredded defiberized pulp fibres to the outside of the drum wall 314, at the station 322. The supply means 324 comprises a housing 326, a pulp sheet inlet 328 leading into the housing 326, a rotatable shredder 330 located within the housing, drive means (not shown) for driving the shredder 330 to rotate it, and an air inlet 331 so that air can enter the housing. Thus, the required air flow inside the housing is generated by the movement of the shredder and the vacuum generating means. As the shredder 330 rotates, it shreds pulp sheet entering the inside of the housing via the inlet 328 into defiberized pulp fibres 332 which are entrained in the air.

The apparatus 310 still further includes a driven bobbin or roll 334 of non-woven fabric located such that a strip 336 of the nonwoven fabric can be fed therefrom directly onto the outside of the drum wall 314, ahead of the supply means 324, as the drum 312 rotates in the direction indicated by arrow 338.

The apparatus 310 still further includes drawing off means (not shown) for drawing off the strip 336 from the drum wall 314 on the other side of the supply means 324. The drawing off means typically comprises a driven wind-up unit, and driven rollers for supporting the strip 336, as required.

The apparatus 310 also includes a spray nozzle 340 for applying a foamed acrylic binder to wads 342 of shredded fibrous pulp formed on the strip 336. A vacuum-generating means 344, generates a vacuum below the strip 336 downstream of the nozzle 340 to spread the foamed binder through the wad and onto the strip 336, Heating means, generally indicated by reference numeral 346, is located above the strip 336, downstream of the vacuum generating means 344 for drying and chemically cross-linking the binder to stabilize the wads 342 and bond them to the strip 336. Typically, the heating means 346 can be steam heated cans. When the wads 342 contain thermoplastic particles, the nozzle 340 and vacuum generating means 344 can be dispensed with, with the wads 342 then being stabilized and bonded directly by passing through or over the heating means 346.

In use, as the drum 312 rotates in the direction of arrow 338, a continuous strip 336 of non-woven fabric is unwound directly from the bobbin 334 so that its surface 336.1 abuts against the outside surface of the drum wall 314. At the station 322, as a result of the vacuum generated by the box 308 and the apertures 320, air passes through the strip 336 only in those areas in alignment with the apertures 320. As the fibres 332 are entrained in the air, the fibres 332 are deposited on surface 336.2 of the strip 336 in the said areas to form the wads 342, while no fibres are deposited elsewhere on that portion of the strip 336 in the forming station 322. The strip 336 with the wads 342 located thereon then pass to the nozzle 340 where the wads 342 are stabilized as hereinbefore described.

After composite products comprising the stabilized wads 342 attached to the strip 336, are formed, they can be laminated to a plastics film backing, eg a polythene film backing, whereafter the polythene film backing and the non-woven fabric can be cut to the same shape (but larger) as the wads. In this fashion soft panty-liners, and the like can be formed. Instead they can be laminated onto an absorbent layer, eg a peatmoss layer, as hereinbefore described, which can then in turn be laminated onto a polythene film backing layer, to form sanitary pads or the like.

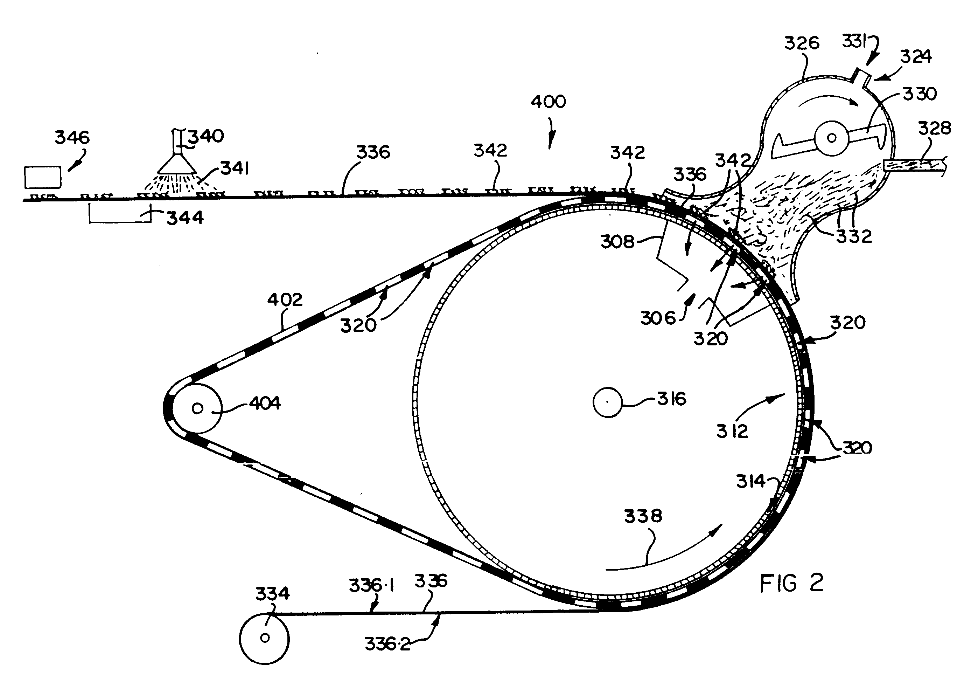

Referring to Figure 2, reference numeral 400 generally indicates apparatus according to another embodiment of the invention, for forming wads,
Parts of the apparatus 400 which are the same or similar to the apparatus 310, are indicated with the same reference numerals.

The apparatus 400 also has a drum 312 having a sieve-like, perforated circular cylindrical wall 314. The drum 312 of the apparatus 400 can be driven by the same drive means as the drum 312 of the apparatus 310. However, the apertures 320 are not provided in the drum wall itself. Instead, the apparatus 400 includes a continuous imperforate belt 402 which passes around the drum 312 as well as around a pulley, idler or roller 404. The position of the idler 404 can be adjusted, thereby to vary the tension in the belt 402. The apertures 320 are provided in the belt 402. Thus, as the drum 312 rotates, the belt 402 continually engages a portion of the drum wall. After the strip 336, with the wads 342 deposited thereon, has been drawn off, the belt disengages from the drum wall 314, and passes around the pulley 404.

The Applicant believes that use of the belt 402 can have advantages. For example, the desired shape of the wads 342 can be varied easily, by merely selecting a belt 402 having apertures 320 of different shapes or sizes. Furthermore, the apparatus is not restricted to sizes and shapes of apertures 320 being selected such that they fit exactly into the drum wall 314. The Applicant further believes that drum blockages on extended runs can be avoided or reduced with the apparatus 400.

The apertures 320 can be of any desired shape, depending on the end use. For example, they may be dogbone-shaped or butterfly-shaped if it is desired to provide panty-liners or sanitary pads respectively.

Referring to Figure 3, a portion of the wall 314 (or the belt 402) is shown. The aperture 320 has a relatively large, rectangular opening 315 with a surrounding "bow-tie" portion 317 with holes 319 therein. The opening 315 provides a region with unrestricted permeability and the portion 317 with its holes 319 provides a region with intermediate permeability.

A wad 342 that is produced with an aperture 320 as shown in Figure 3, is shown in Figure 4. Thus, the wad 342 has a substantially "bow-tie" external profile with a central rectangular portion 319 and a border 321. The central portion 319 is thicker than the border 321, as more air passes through that area of the strip 336 in alignment with the opening 315 than that in alignment with the portion 317, and therefore more fibres are collected in the middle than on the sides.

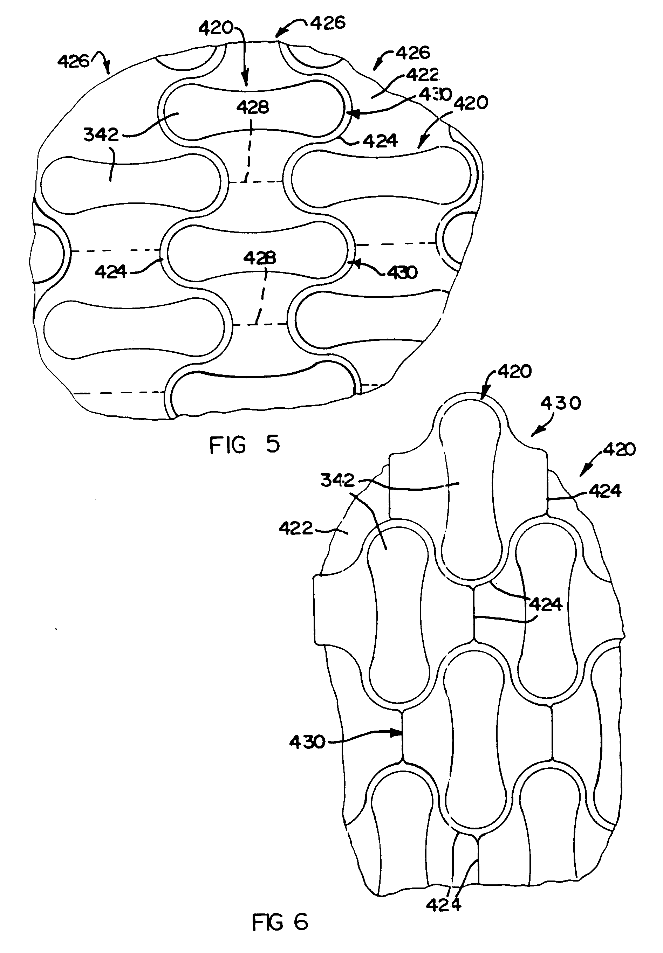

It will be appreciated that with the apparatus 310, 400, the drum 312 can be made of any desired length, so that a number of wads can be formed side-by-side simultaneously. The apertures 320 need then naturally not be aligned with each other along the length of the drum wall 314, but can be staggered so that rows 420 (see Figures 5 and 6) of composite products, each comprising a layer of non-woven fabric and wads 342 of stabilized defiberized pulp particles located thereon as described, and a layer 422 of fluid-impervious material, eg a plastics film backing, laminated thereto, are produced. In a cutting station (not shown) these rows can then be separated from one another by cutting along more-or-less sinusoidal, generally longitudinally extending, cut lines 424, into strips 426 of composite product. The strips 426 can then be cut along transversely extending cut lines 428 to produce individual composite products 430 comprising pads with side flaps. In the case of the arrangement as shown in Figure 6, the individual composite products 430 are formed once the cuts along the cut lines 424 have been effected, so that the additional cuts along transverse cut lines are not required.

## Claims

1. A method of forming a wad (342) of particulate material (332), the wad having a predetermined shape, which includes
providing a permeable carrier (336) which has first (336.2) and second (336.1) opposed surfaces;
providing a forming screen (314,402) which also has first and second opposed surfaces and which is substantially impermeable except for a forming zone (320) which has the said shape and which is permeable;
positioning the carrier and the screen adjacent one another;
generating a pressure differential across the carrier and the screen, there being a higher pressure at the first surface of the carrier;
introducing the particulate material into the space adjacent the first surface of the carrier, which particulate material is carried by the fluid stream; and
separating the carrier and the screen, characterised thereby that the screen is located with its first surface adjacent the second surface of the carrier; and
the stream of fluid flows through the carrier substantially only in an area that is aligned with the forming zone of the screen such that the particulate material is deposited on the first surface of the carrier in the said area to form the wad.

2. The method claimed in Claim 1, characterised thereby that the forming zone is an aperture (320) in the screen.

3. The method claimed in Claim 1, characterised thereby that the forming zone has a region of substantially high permeability (315) and a region of intermediate permeability (320), with more particulate material being deposited on an area of the carrier in alignment with the high permeability region and less being deposited on an area in alignment with the intermediate permeability region.

4. The method claimed in Claim 1, characterised thereby that the screen has a plurality of forming zones that are spaced apart, and the carrier and the screen are moved together through a pressurizing and introducing station (322) where the fluid stream is caused to flow through the carrier only in those areas that are aligned with forming zones, thereby depositing the particulate material on the carrier and successively forming the wads thereon.

5. The method claimed in Claim 4, characterised thereby that the carrier and the screen are moved continuously through the pressurizing and introducing station.

6. The method claimed in Claim 4, characterised thereby that a plurality of forming zones are located together in the pressurizing and introducing station.

7. The method claimed in Claim 4, characterised thereby that the screen is endless.

8. The method claimed in Claim 1, characterised thereby that the carrier is a strip of non-woven fabric.

9. The method claimed in Claim 1, characterised thereby that the particulate material is fibrous.

10. The method claimed in Claim 7, characterised thereby that the screen comprises a cylindrical wall (314) of a drum.

11. The method claimed in Claim 7, characterised thereby that the screen comprises a belt (402) which is supported, in the pressurizing and introducing station, by a drum (312) having a sieve-like cylindrical wall.

12. The method claimed in Claim 1, characterised thereby that the carrier and the screen are in contact with one another.

13. An apparatus (310, 400) for forming a wad (342) of particulate material (332), the wad having a predetermined shape, which includes
a forming screen (314, 402) which has first and second opposed surfaces and which is substantially impermeable except for a forming zone (320) which has the said shape and which is permeable;
a supply means (334) for supplying a permeable carrier, which also has first (336.2) and second (336.1) opposed surfaces;
a pressure differential generating means (308) for generating, in use, a pressure differential across the carrier and the screen, there being a higher pressure at the first surface of the carrier;
introducing means (324) for introducing the particulate material, in use, into the space adjacent the first surface of the carrier, such that the particulate material is carried by the fluid stream to be deposited on the first surface of the carrier; and
separating means for separating, in use, the carrier and the screen, characterised thereby that the screen is located, in use, with its first surface adjacent the second surface of the carrier such that, in use, the stream of fluid flows through the carrier substantially only in an area that is aligned with the forming zone of the screen and the particulate material is deposited on the first surface of the carrier in the said area to form the wad.

14. The apparatus claimed in Claim 13, characterised thereby that it includes the carrier.

15. The apparatus claimed in Claim 13, characterised thereby that the screen has an aperture (320) which defines the forming zone.

16. The apparatus claimed in Claim 13, characterised thereby that the forming zone has a region of substantially high permeability (315) and a region of intermediate permeability (320), such that, in use, more particulate material is deposited on an area of the carrier in alignment with the high permeability region and less is deposited on an area of the carrier in alignment with the intermediate permeability region.

17. The apparatus claimed in Claim 13, characterised thereby that the screen has a plurality of forming zones that are spaced apart and the pressure differential generating means and the introducing means constitute a pressurizing and introducing station (322); and which includes a moving means (352, 354) for moving, in use, the carrier and the screen together through the said station, such that the fluid stream is caused to flow through the carrier only in those areas that are aligned with forming zones, thereby depositing the particulate material on the carrier and successively forming the wads thereon.

18. The apparatus claimed in Claim 17, characterised thereby that the moving means moves the carrier and the screen continuously through the pressurizing and introducing station.

19. The apparatus claimed in Claim 17, characterised thereby that a plurality of the forming zones are located together in the pressurizing and introducing station

20. The apparatus claimed in Claim 17, characterised thereby that the screen is endless.

21. The apparatus claimed in Claim 14, characterised thereby that the carrier is a roll of a non-woven fabric.

22. The apparatus claimed in Claim 13, characterised thereby that the particulate material is fibrous.

23. The apparatus claimed in Claim 20, characterised thereby that the screen comprises a cylindrical wall (314) of a drum.

24. The apparatus claimed in Claim 20, characterised thereby that the screen comprises a belt (402), and which includes a support drum (312) having a sieve-like cylindrical wall for supporting the belt and the carrier at the pressurizing and introducing station.

25. The apparatus claimed in Claim 13, characterised thereby that the supply means supplies the carrier, in use, in contact with the screen.

26. The apparatus claimed in Claim 13, characterised thereby that the pressure differential generating means (308) comprises a vacuum generating means located adjacent the second surface of the screen.

27. The apparatus claimed in Claim 22, characterised thereby that the introducing means includes a shredder (330) for shredding a sheet (328) of fibrous material.

## Patentansprüche

1. Verfahren zum Formen eines Kissens (342) aus partikelförmigem Material (332), wobei das Kissen eine vorbestimmte Form hat, umfassend:
Vorsehen eines durchlässigen Trägers (336), der erste (336.2) und zweite (336.1) gegenüberliegende Oberflächen hat;
Vorsehen eines formenden Siebes (314, 402), das ebenfalls erste und zweite gegenüberliegende Oberflächen hat, und das im wesentlichen undurchlässig ist, mit Ausnahme einer Formgebungszone (320) die die genannte Form hat und durchlässig ist;
Anordnen des Trägers und des Siebes benachbart zueinander;
Erzeugen einer Druckdifferenz quer durch den Träger und das Sieb, wobei ein höherer Druck an der ersten Oberfäche des Trägers herrscht;
Einführen des partikelförmigen Materials in den Raum benachbart zur ersten Oberfläche des Trägers, welches partikelförmige Material durch den Fluidstrom getragen wird; und
Trennen des Trägers und des Siebes, dadurch gekennzeichnet, daß das Sieb mit seiner ersten Oberfläche benachbart zur zweiten Oberfläche des Trägers angeordnet ist; und
der Fluidstrom im wesentlichen lediglich in einem Bereich durch den Träger strömt, der mit der Formgebungszone des Siebes derart ausgerichtet ist, daß das partikelförmige Material auf der ersten Oberfläche des Trägers in dem Bereich zur Bildung des Kissens abgelegt wird.

2. Verfahren nach Anspruch 1, dadurch gekennzeichnet, daß die Formgebungszone eine Öffnung (320) in dem Sieb ist.

3. Verfahren nach Anspruch 1, dadurch gekennzeichnet, daß die Formgebungszone einen Bereich (315) im wesentlichen hoher Durchlässigkeit und einen Bereich (320) mittlerer Durchlässigkeit hat, wobei mehr partikelförmiges Material auf einen Bereich des Trägers in Ausrichtung mit dem Bereich hoher Durchlässigkeit und weniger auf einem Bereich in Ausrichtung mit dem Bereich mittlerer Durchlässigkeit abgelegt wird.

4. Verfahren nach Anspruch 1, dadurch gekennzeichnet, daß das Sieb eine Mehrzahl beabstandeter Formgebungszonen hat, und der Träger und das Sieb zusammen durch eine Druckausübungs- und Einführstation (322) bewegt werden, wo der Fluidstrom dazu veranlaßt wird, lediglich in solchen Bereichen durch den Träger zu strömen, die mit Formgebungszonen ausgerichtet sind, wodurch das partikelförmige Material auf den Träger abgelegt und die Kissen aufeinanderfolgend geformt werden.

5. Verfahren nach Anspruch 4, dadurch gekennzeichnet, daß der Träger und das Sieb kontinuierlich durch die Druck- und Einführstation bewegt werden.

6. Verfahren nach Anspruch 4, dadurch gekennzeichnet, daß eine Mehrzahl von Formgebungszonen zusammen in der Druck- und Einführstation angeordnet sind.

7. Verfahren nach Anspruch 4, dadurch gekennzeichnet, daß das Sieb endlos ist.

8. Verfahren nach Anspruch 1, dadurch gekennzeichnet, daß der Träger ein Streifen aus nicht gewebtem Stoff ist.

9. Verfahren nach Anspruch 1, dadurch gekennzeichnet, daß das partikelförmige Material faserförmig ist.

10. Verfahren nach Anspruch 7, dadurch gekennzeichnet, daß das Sieb eine zylindrische Wand (314) einer Trommel umfaßt.

11. Verfahren nach Anspruch 7, dadurch gekennzeichnet, daß das Sieb ein Band (402) umfaßt, das in der Druck- und Einführstation durch eine Trommel (312) getragen ist, die eine siebartige zylindrische Wand hat.

12. Verfahren nach Anspruch 1, dadurch gekennzeichnet, daß der Träger und das Sieb sich berühren.

13. Vorrichtung (310, 400) zum Formen eines Kissens (342) aus partikelförmigem Material (332), wobei das Kissen eine vorbestimmte Form hat, mit
einem formenden Sieb (314, 402), das erste und zweite gegenüberliegende Oberflächen hat, und das im wesentlichen undurchlässig ist, mit Ausnahme einer Formgebungszone (320) welche die besagte Gestalt hat und durchlässig ist;
einer Zufuhreinrichtung (334) zum Zuführen eines durchlässigen Trägers, der ebenfalls erste (336.2) und zweite (336.1) gegenüberliegende Oberflächen hat;
einer Druckdifferenzerzeugungseinrichtung (308) zum Erzeugen einer Druckdifferenz im Betrieb quer durch den Träger und das Sieb, so daß ein höherer Druck an der ersten Oberfäche des Trägers herrscht;
einer Einführvorrichtung (324) die im Betrieb das partikelförmigen Material in den der ersten Oberfläche benachbarten Raum einführt derart, daß das partikelförmige Material durch den Fluidstrom zum Ablegen auf der ersten Oberfläche des Trägers mitgeführt wird; und einer Trenneinrichtung die im Betrieb den Träger und das Sieb trennt, dadurch gekennzeichnet, daß das Sieb im Betrieb mit seiner ersten Oberfläche benachbart zur zweiten Oberfläche des Trägers derart angeordnet ist, daß der Fluidstrom im Einsatz im wesentlichen lediglich in einem Bereich durch den Träger strömt, der mit der Formgebungszone des Siebes ausgerichtet ist, und das partikelförmige Material auf der ersten Oberfläche des Trägers in diesem Bereich zum Formen des Kissens abgelegt wird.

14. Vorrichtung nach Anspruch 13, dadurch gekennzeichnet, daß sie den Träger umfaßt.

15. Vorrichtung nach Anspruch 13, dadurch gekennzeichnet, daß das Sieb eine Öffnung (320) hat, die die Formgebungszone bestimmt.

16. Vorrichtung nach Anspruch 13, dadurch gekennzeichnet, daß die Formgebungszone einen Bereich (315) im wesentlichen hoher Durchlässigkeit und einen Bereich (320) mittlerer Durchlässigkeit derart hat, daß im Betrieb mehr partikelförmiges Material auf einem Bereich des Trägers in Ausrichtung mit dem Bereich hoher Durchlässigkeit und weniger auf einen Bereich des Trägers in Ausrichtung mit dem Bereich mittlerer Durchlässigkeit abgelegt wird.

17. Vorrichtung nach Anspruch 13, dadurch gekennzeichnet, daß das Sieb eine Mehrzahl von beabstandeten Formgebungszonen hat und die Druckdifferenzerzeugungseinrichtung und die Einführeinrichtung eine Druck- und Einführstation (322) bilden, die eine Antriebsvorrichtung (352, 354) durch die im Betrieb der Träger und das Sieb zusammen durch die genannte Station bewegbar sind, derart, daß der Fluidstrom dazu veranlaßt wird, durch den Träger ausschließlich in solchen Bereichen zu strömen, die mit Formgebungszonen ausgerichtet sind, wodurch das partikelförmige Material auf den Träger abgelegt wird und die Kissen aufeinanderfolgend darauf ausgeformt werden.

18. Vorrichtung nach Anspruch 17, dadurch gekennzeichnet, daß die Antriebsvorrichtung den Träger und das Sieb kontinuierlich durch die Druck- und Einführstation bewegt.

19. Vorrichtung nach Anspruch 17, dadurch gekennzeichnet, daß eine Mehrzahl der Formgebungszonen zusammen in der Druck- und Einführstation angeordnet ist.

20. Vorrichtung nach Anspruch 17, dadurch gekennzeichnet, daß das Sieb endlos ist.

21. Vorrichtung nach Anspruch 14, dadurch gekennzeichnet, daß der Träger eine Rolle aus nicht gewebtem Stoff ist.

22. Vorrichtung nach Anspruch 13, dadurch gekennzeichnet, daß das partikelförmige Material faserförmig ist.

23. Vorrichtung nach Anspruch 20, dadurch gekennzeichnet, daß das Sieb eine zylindrische Wand (314) einer Trommel umfaßt.

24. Vorrichtung nach Anspruch 20, dadurch gekennzeichnet, daß das Sieb ein Band (402) umfaßt und eine Tragtrommel (312) vorgesehen ist, die eine siebartige zylindrische Wand zum Tragen des Bandes und des Trägers in der Druck- und Einführstation hat.

25. Vorrichtung nach Anspruch 13, dadurch gekennzeichnet, daß die Zufuhreinrichtung im Betrieb den Träger und das Sieb in Berührung bringt.

26. Vorrichtung nach Anspruch 13, dadurch gekennzeichnet, daß die Druckdifferenzerzeugungseinrichtung (308) eine Vakuumerzeugungseinrichtung umfaßt, die der zweiten Oberfläche des Siebes benachbart angeordnet ist.

27. Vorrichtung nach Anspruch 22, dadurch gekennzeichnet, daß die Einführvorrichtung einen Schredder (330) zum Schreddern einer Schicht (328) aus faserförmigem Material umfaßt.

## Revendications

1. Procédé de formation d'un coussin (342) de matériau particulaire (332), le coussin ayant une forme prédéterminée, qui comporte les étapes consistant à:
fournir un support (336) perméable qui a une première et une seconde surfaces opposées (336.2, 336.1),
fournir un écran de formation (314, 402) qui a aussi une première et une seconde surfaces opposées et qui est pratiquement imperméable à l'exception d'une zone de formation (320) qui a ladite forme et qui est perméable,
positionner le support et l'écran adjacents l'un à l'autre,
créer un différentiel de pression à travers le support et l'écran, une pression plus élevée existant au niveau de la première surface du support,
introduire le matériau particulaire à l'intérieur de l'espace adjacent à la première surface du support, lequel matériau particulaire est transporté par un flux de fluide, et
séparer le support et l'écran,
caractérisé en ce que l'écran est situé en ayant sa première surface adjacente à la seconde surface du porteur, et
le flux de fluide s'écoule à travers le support pratiquement uniquement dans une aire qui est alignée avec la zone de formation de l'écran de telle sorte que le matériau particulaire soit déposé sur la surface du support dans ladite aire destinée à former le coussin.

2. Procédé selon la revendication 1, caractérisé en ce que la zone de formation est une ouverture (320) agencée dans l'écran.

3. Procédé selon la revendication 1, caractérisé en ce que la zone de formation comporte une région (315) ayant une perméabilité très élevée et une région (320) ayant une perméabilité intermédiaire, plus de matériau particulaire étant déposé sur une aire du porteur alignée avec la région à perméabilité élevée et moins de matériau particulaire étant déposé sur une aire alignée avec la région à perméabilité intermédiaire.

4. Procédé selon la revendication 1, caractérisé en ce que l'écran comporte plusieurs zones de formation qui sont écartées les unes des autres et le support et l'écran sont déplacés ensemble à travers un poste de mise sous pression et d'introduction (322) dans lequel le flux de fluide est amené à s'écouler à travers le support uniquement dans les aires qui sont alignées avec les zones de formation, en déposant ainsi le matériau particulaire sur le support et en formant les coussins de manière successive sur celui-ci.

5. Procédé selon la revendication 4, caractérisé en ce que le support et l'écran sont déplacés de manière continue à travers le poste de mise sous pression et d'introduction.

6. Procédé selon la revendication 4, caractérisé en ce que plusieurs zones de formation sont situées ensemble dans le poste de mise sous pression et d'introduction.

7. Procédé selon la revendication 4, caractérisé en ce que l'écran est sans fin,

8. Procédé selon la revendication 1, caractérisé en ce que le support est une bande de tissu non tissé.

9. Procédé selon la revendication 1, caractérisé en ce que le matériau particulaire est fibreux.

10. Procédé selon la revendication 7, caractérisé en ce que l'écran est constitué d'une paroi cylindrique (314) d'un tambour.

11. Procédé selon la revendication 7, caractérisé en ce que l'écran est constitué d'une courroie (402) qui est supportée, dans le poste de mise sous pression et d'introduction, par un tambour (312) ayant une paroi cylindrique analogue à un tamis.

12. Procédé selon la revendication 1, caractérisé en ce que le support et l'écran sont en contact l'un avec l'autre.

13. Dispositif (310, 400) pour former un coussin (342) de matériau particulaire (332), le coussin ayant une forme prédéterminée, qui comporte
un écran de formation (314, 402) qui a une première et une seconde surfaces opposées et qui est pratiquement imperméable à l'exception d'une zone de formation (320) qui a ladite forme et qui est perméable,
des moyens d'alimentation (334) pour alimenter un support perméable, qui a aussi une première et une seconde surfaces opposées (336.2, 336.1),
des moyens (308) pour créer un différentiel de pression pour engendrer en utilisation un différentiel de pression à travers le support et l'écran, une pression plus élevée existant au niveau de la première surface du support,
des moyens d'introduction (324) destinés à introduire le matériau particulaire, en utilisation, à l'intérieur de l'espace adjacent à la première surface du support, de telle sorte que le matériau particulaire soit transporté par le flux de fluide pour être déposé sur la première surface du support, et
des moyens de séparation pour séparer en utilisation, le support et l'écran, caractérisé en ce que l'écran est situé, en utilisation, en ayant sa première surface adjacente à la seconde surface du support de telle sorte qu'en utilisation le flux de fluide s'écoule à travers le support pratiquement uniquement dans une aire qui est alignée avec la zone de formation de l'écran et le matériau particulaire est déposé sur la première surface du support dans ladite aire pour former le coussin.

14. Dispositif selon la revendication 13, caractérisé en ce qu'il comporte le support.

15. Dispositif selon la revendication 13, caractérisé en ce que l'écran a une ouverture (320) qui définit la zone de formation.

16. Dispositif selon la revendication 13, caractérisé en ce que la zone de formation a une région (315) de perméabilité très élevée et une région (320) de perméabilité intermédiaire de telle sorte que, en utilisation, plus de matériaux particulaires soient déposés sur une aire du support alignée avec la région à perméabilité élevée et moins de matériaux soient déposés sur une aire du support alignée avec la région à perméabilité intermédiaire.

17. Dispositif selon la revendication 13 caractérisé en ce que l'écran comporte plusieurs zones de formations qui sont espacées et les moyens de création de différentiel de pression et les moyens d'introduction constituent un poste (322) de mise sous pression et d'introduction, et qui comporte des moyens de déplacement (352, 354) destinés à déplacer, en utilisation, le support et l'écran ensemble à travers ledit poste, de telle sorte que le flux de fluide est amené à s'écouler à travers le support uniquement dans les aires qui sont alignées avec les zones de formation, déposant ainsi le matériau particulaire sur le support et formant les coussins de manière successive sur celui-ci.

18. Dispositif selon la revendication 17, caractérisé en ce que les moyens de déplacement déplacent le support et l'écran de manière continue à travers le poste de mise sous pression et d'introduction.

19. Dispositif selon la revendication 17, caractérisé en ce que plusieurs zones de formation sont situées ensemble dans le poste de mise sous pression et d'introduction,

20. Dispositif selon la revendication 17, caractérisé en ce que l'écran est sans fin.

21. Dispositif selon la revendication 14, caractérisé en ce que le support est un rouleau de tissu non tissé.

22. Dispositif selon la revendication 13, caractérisé en ce que le matériau particulaire est fibreux.

23. Dispositif selon la revendication 20, caractérisé en ce que l'écran est constitué de la paroi cylindrique (314) d'un tambour.

24. Dispositif selon la revendication 20, caractérisé en ce que l'écran est constitué d'une courroie (102), et en ce qu'il comporte un tambour de support (312) ayant une paroi cylindrique analogue à un tamis pour supporter la courroie et le support au niveau du poste de mise sous pression et d'introduction.

25. Dispositif selon la revendication 13, caractérisé en ce que les moyens d'alimentation alimentent le support, en utilisation, en contact avec l'écran.

26. Dispositif selon la revendication 13, caractérisé en ce que les moyens (308) de création de différentiel de pression sont constitués de moyens créant un vide adjacent à la seconde surface de l'écran.

27. Dispositif selon la revendication 22, caractérisé en ce que les moyens d'introduction comportent un déchiqueteur (330) pour déchiqueter une feuille (328) de matériaux fibreux.
